# FASCICULE DE BREVET EUROPEEN

(11) **EP 0 703 217 B1**
(45) Date de publication et mention de la délivrance du brevet: **11.09.1996**
(21) Numéro de dépôt: 95401767.9
(22) Date de dépôt: 25.07.1995
(51) Int. Cl.: C07C 271/22, A61K 7/48, A61K 47/18, C10M 115/08, C09D 7/00, C10L 1/22

(54) **Esters alkyliques fluorés ayant une fonction uréthane, leur préparation, leur utilisation pour épaissir un milieu non aqueux, les milieux et les compositions les comprenant**
Eine Urethangruppe enthaltende, fluorierte Alkylester, ihre Herstellung, ihre Verwendung als Verdickungsmittel in nicht wässrigen Medien, und diese enthaltende Medien und Zusammensetzungen
Fluorinated alkylesters containing a urethane group, their preparation their use as thickeners in non-aqueous media and media and compositions containing them

(30) Priorité: 21.09.1994 FR 9411266
(43) Date de publication de la demande: 27.03.1996
(73) Titulaire: L'OREAL, F-75008 Paris (FR)
(72) Inventeur: Bollens, Eric, F-94410 Saint-Maurice (FR); Mahieu, Claude, F-75017 Paris (FR); Philippe, Michel, F-91230 Wissous (FR)
(74) Mandataire: Miszputen, Laurent

(56) Documents cités:
- EP-A- 0 563 978
- WO-A-93/11103
- FR-A- 2 647 445

## Description

L'invention se rapporte à de nouveaux composés fluorés, à leur préparation, leur utilisation, ainsi qu'aux milieux et aux compositions les comprenant.

La demande de brevet FR 2647445 décrit des composés de la famille des acides 11-N-alkyloxycarbonylamino-undécanoïques et leurs esters non fluorés, ainsi que leur utilisation en tant qu'agents épaississants notamment de milieux non aqueux. Ces composés présentent le désavantage de ne pas permettre l'obtention de solutions transparentes. De plus on constate une certaine instabilité entraînant l'apparition de cristaux dans les solutions épaissies les contenant.

C'est pour chercher à éliminer les désavantages de ces composés que la demanderesse a recherché de nouveaux composés présentant au moins les mêmes propriétés épaississantes.
C'est ainsi que la demanderesse a mis au point de nouveaux composés à chaînes fluorées utilisables entre autres, en tant qu'agent épaississant.
Ces composés sont utilisables de préférence avec des milieux non aqueux.

L'invention a donc pour objet des composés fluorés répondant à la formule générale : 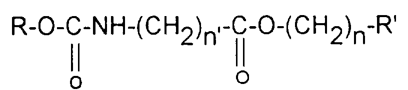 dans laquelle
. R représente un radical hydrocarboné linéaire ou ramifié, ayant de 8 à 22 atomes de carbone,
. R' représente un radical perfluorocarboné, linéaire ou ramifié, ayant de 4 à 20 atomes de carbone,
. n est compris entre 0 et 4, et n' est compris entre 1 et 11.

Par radical hydrocarboné, on entend tout radical hydrocarboné saturé ou insaturé ayant le nombre d'atomes de carbone requis.
Par radical perfluorocarboné, on entend tout radical carboné dans lequel tous les atomes d'hydrogène ont été remplacés par des atomes de fluor.

Selon un mode de réalisation particulier de l'invention, R contient de préférence entre 8 et 16 atomes de carbone.
Selon un autre mode de réalisation de l'invention R' contient de préférence entre 4 et 10 atomes de carbone.
De préférence R' est un radical perfluorocarboné saturé.

On peut citer comme composés fluorés de formule (I) :
. le 11-N-hexadécyloxycarbonylamino-undécanoate de 2-F-octyl éthyle ;
. le 11-N-hexadécyloxycarbonylamino-undécanoate de 2-F-hexyl éthyle ;
. le 2-N-hexadécyloxycarbonylamino-éthanoate de 2-F-octyl éthyle ;
. le 2-N-hexadécyloxycarbonylamino-éthanoate de 2-F-hexyl éthyle ;
. le 6-N-hexadécyloxycarbonylamino-hexanoate de 2-F-octyl éthyle ;
. le 6-N-hexadécyloxycarbonylamino-hexanoate de 2-F-hexyl éthyle ;
. le 11-N-docosyloxycarbonylamino-undécanoate de 2-F-octyl éthyle ;
. 11-N-docosyloxycarbonylamino-undécanoate de 2-F-hexyl éthyle ;
. le 8-N-hexadécyloxycarbonylamino-octanoate de 2-F-octyl éthyle ;
. le 8-N-hexadécyloxycarbonylamino-octanoate de 2-F-hexyl éthyle.

Un deuxième objet de l'invention est un procédé de préparation des composés de formule (I) tels que définis ci-dessus.
Ce procédé est caractérisé par le fait que l'on fait réagir, dans une réaction d'estérification classique connue de l'homme du métier, en présence d'un solvant et en milieu acide, un composé de la famille des acides ω-N-alkyloxycarbonylamino-alkylénecarboxyliques, répondant à la formule : 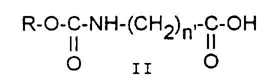 avec un alcool fluoré répondant à la formule :

R'-(CH₂)ₙ-OH

dans lesquelles
. R représente un radical hydrocarboné linéaire ou ramifié, ayant de 8 à 22 atomes de carbone,
. R' représente un radical perfluorocarboné, linéaire ou ramifié, ayant de 4 à 20 atomes de carbone,
. n est compris entre 0 et 4, et n' est compris entre 1 et 11.

Selon un mode de réalisation particulier de l'invention, R contient de préférence entre 8 et 16 atomes de carbone.
Selon un autre mode de réalisation de l'invention R' contient de préférence entre 4 et 10 atomes de carbone.
De préférence R' est un radical perfluorocarboné saturé.

Le solvant utilisé peut être un solvant neutre classique de ce type de réaction, bien connu de l'homme du métier. On peut citer par exemple le toluène.

Un troisième objet de l'invention est un autre procédé de préparation des composés de formule (I) tels que définis ci-dessus.
Ce procédé est caractérisé par le fait que l'on fait réagir dans une réaction d'estérification classique, connue de l'homme du métier, en présence d'un solvant et éventuellement en catalyse basique, un dérivé activé de la famille des acides ω-N-alkyloxycarbonylamino-alkylénecarboxyliques, répondant à la formule : 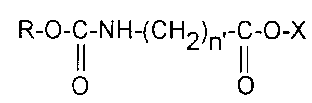 avec un alcool fluoré répondant à la formule :

R'-(CH₂)ₙ-OH

dans lesquelles
. R représente un radical hydrocarboné linéaire ou ramifié, ayant de 8 à 22 atomes de carbone,
. R' représente un radical perfluorocarboné, linéaire ou ramifié, ayant de 4 à 20 atomes de carbone,
. n est compris entre 0 et 4, n' est compris entre 1 et 11, et
. X représente un groupement activant comme notamment un chlore, un fluor, un azole comme l'imidazole ou un groupement OCOOR'', dans lequel R'' est un radical hydrocarboné ayant de 2 à 4 atomes de carbone.

Selon un mode de réalisation particulier de l'invention, R contient de préférence entre 8 et 16 atomes de carbone.

Selon un autre mode de réalisation de l'invention R' contient de préférence entre 4 et 10 atomes de carbone.
De préférence R' est un radical perfluorocarboné saturé.

Un quatrième objet de l'invention concerne l'utilisation des composés fluorés de formule I pour épaissir un milieu non aqueux.
Plus particulièrement ils trouvent une application en tant qu'agent épaississant et/ou gélifiant de liquides huileux ou de milieux non aqueux à large spectre de polarité. Par milieux à large spectre de polarité on entend aussi bien des milieux apolaires comme par exemple l'huile de vaseline que des milieux polaires comme les composés à fonction alcool.
On peut, par exemple, utiliser ces agents épaississants, et/ou les milieux épaissis les contenant, dans les domaines de la cosmétique, de la pharmacie, des peintures et vernis, des lubrifiants, des combustibles et de l'industrie alimentaire.
Ces composés ont des qualités épaississantes remarquables. Cette propriété a pour conséquence de permettre l'utilisation de faibles quantités de ce type de composé dans les milieux à épaissir.
Ces nouveaux composés permettent d'obtenir le plus souvent, des milieux épaissis transparents qui présentent une homogénéité stable dans le temps.
Ces nouveaux composés, en raison de leur caractère non ionique, confèrent également aux compositions les comprenant une grande douceur lorsqu'elles sont utilisées.
Le composé fluoré peut être utilisé à de très faibles concentrations. En général elles sont comprises entre 0,01% et 25% et de préférence entre 0,1% et 10%, en poids par rapport au poids total de milieu épaissi.
L'utilisation de ces composés peut également permettre l'obtention d'un milieu gélifié.
L'agent épaississant peut être un mélange de composés de formule I, pour lesquels R et/ou R' sont des chaînes de longueurs différentes.
L'épaississement du milieu peut s'effectuer par solubilisation complète du composé fluoré de formule I, à une température comprise entre 20°C et 80°C, puis en laissant reposer le mélange jusqu'à ce qu'il soit totalement épaissi.

Un cinquième objet de l'invention concerne un milieu non aqueux comprenant au moins un composé répondant à la formule I.
Dans ce milieu, le composé fluoré de formule I peut jouer le rôle d'agent épaississant.

Dans ce milieu, l'agent épaississant peut être utilisé à des concentrations comprises entre 0.01% et 25% et de préférence entre 0.1% et 10%, en poids par rapport au poids total de milieu épaissi.
Ce milieu peut être composé de liquide huileux et/ou de milieux non aqueux à large spectre de polarité. Ce milieu peut constituer, ou être constituant, de compositions cosmétiques ou pharmaceutiques, de peintures et de vernis, de lubrifiants, de combustibles ou encore de produits alimentaires.
Lorsque le milieu non aqueux est par exemple une composition cosmétique il peut comprendre les ingrédients habituellement utilisés dans ce type de préparations, comme cela est défini ci-dessous.

Un sixième objet de l'invention concerne une composition comprenant au moins un milieu non aqueux comprenant au moins un composé fluoré répondant à la formule I.
Dans cette composition, le composé fluoré de formule I peut jouer le rôle d'agent épaississant du milieux non aqueux.
Ce type de compositions peut être destinée à la cosmétique, à la pharmacie, aux peintures et vernis, aux lubrifiants, aux combustibles ou encore à l'industrie alimentaire.
En cosmétique ou en pharmacie, ce type de compositions peut, entre autre, se présenter sous forme de sticks, de laits ou de vernis.
Elles comprennent alors, les ingrédients habituellement utilisés en cosmétique ou en pharmacie, pour ce type de préparation. Ainsi elles peuvent comprendre au moins un additif choisi parmi les alcools gras, les épaississants, les esters d'acides gras, les esters d'acides gras et de glycérol, les silicones (volatiles ou non, fonctionnalisés ou non), les tensioactifs, les parfums, les conservateurs, les filtres solaires, les protéines, les vitamines, les polymères, les huiles organiques ou inorganiques et tout autre additif classiquement utilisé dans les domaines cosmétique ou pharmaceutique.
Toutes ces compositions sont préparées selon les méthodes usuelles connues de l'homme de l'art.
On va maintenant donner à titre d'illustration des exemples de préparation de composés fluorés, répondant à la formule (I), ainsi que des exemples de compositions les comprenant. Ces exemples ne sauraient limiter en aucune façon la portée de l'invention.

### Exemple 1 : Synthèse du 11-N-hexadécyloxycarbonylamino-undécanoate de 2-F-octyl éthyle

Dans un réacteur de 1 litre équipé d'un appareil de Dean et Stark, on introduit :
. 39 g (84 mmoles), de 2-F-octylethanol*
. 37 g (80 mmoles) d'acide 11-N-hexadécyloxycarbonylamino-undécanoïque
. 440 g de toluène
. 2,25 g d'acide para toluène sulfonique.

* : commercialisé par ATOCHEM sous le nom de FORALKYL EOH 8

Le mélange est porté au reflux du solvant pendant 16 heures. On concentre la solution pour obtenir un solide blanc qui est filtré et recristallisé dans l'éther diisopropylique.
On obtient ainsi 38 g (41,5 mmoles) de 11-N-hexadécyloxycarbonylamino-undécanoate de 2-F-octyl éthyle sous la forme d'un solide blanc, ce qui corres-pond à un rendement de 52%.

### Analyse :

Point de fusion: 98°C (Mettler FP89)
CCM : Gel de silice 60, éluant CH₂Cl₂: Rf = 0,6.
Spectre de R.M.N. ¹³C : conforme
Spectre de masse : conforme

| Analyse élémentaire : | | | | |
|---|---|---|---|---|
| | % C | % H | % N | % F |
| Théorique | 49,83 | 6,38 | 1,53 | 35,27 |
| Expérimentale | 49,95 | 6,36 | 1,50 | 35,25 |

L'analyse confirme l'obtention du produit attendu.

### Exemple 2 : Synthèse du 11-N-hexadécyloxycarbonylamino-undécanoate de 2-F- hexyl éthyle

Dans un réacteur de 0,5 litre équipé d'un appareil de Dean et Stark, on introduit:
. 8,1 g (22 mmoles), de 2-F-hexyléthanol*
. 9,38 g (20 mmoles) d'acide 11-N-hexadécyloxycarbonylamino-undécanoïque
. 240 g de toluène
. 6 g d'acide paratoluènesulfonique.

* : commercialisé par ATOCHEM sous le nom de FORALKYL EOH 8

Le mélange est porté au reflux du solvant pendant 16 heures. On concentre la solution pour obtenir un résidu qui est qui est repris par un minimum de dichlorométhane et chromatographié sur colonne de gel de silice.

On obtient finalement 9,5 g, (11,6 mmoles) de 11-N-hexadécyloxycarbonyl-amino-undécanoate de 2-F-hexyl éthyle pour un rendement de 58 %.

### Analyse :

Point de fusion: 86,9°C (Mettler FP89)
CCM : Gel de silice 60, éluant CHCl₃ : Rf = 0,2
Spectre de R.M.N. ¹³C : conforme
Spectre de masse : conforme

| Analyse élémentaire : | | | | |
|---|---|---|---|---|
| | % C | % H | % N | % F |
| Théorique | 53,00 | 7,17 | 1,72 | 30,27 |
| Expérimentale | 53,53 | 7,38 | 1,87 | 29,52 |

L'analyse confirme l'obtention du produit attendu.

### Exemple 3 : Synthèse du 2-N-hexadécyloxycarbonylamino-éthanoate de 2-F-octyl éthyle

### Mode opératoire :

Dans un ballon de 250 ml équipé d'un appareil de Dean Stark surmonté d'un réfrigérant sont introduits 16,7 g (0,036 mole) de 2-F-octyl éthanol, 10,31 g (0,03 mole) de N-hexadécyloxycarbonyl glycine ainsi que 90 ml de toluène en présence de 0,86 g d'acide paratoluène sulfonique. Le milieu réactionnel est chauffé pendant 16 heures au reflux du toluène (130°C). 10 g de silice 60H sont ajoutés à 80°C et cette température est maintenue pendant 5 min, puis le mélange est filtré sur fritté n°4, lavé avec 30 ml de toluène chaud. Le filtrat fige dans la fiole à vide, il est filtré sur fritté n°4, séché sous-vide, et recristallisé dans 50 ml d'éther isopropylique, le produit obtenu est une poudre blanche ;
m = 16 g ; rendement = 68 %.

### Analyses :

F(FP85 METTLER) : 77°C
CCM Gel de silice 60 ; éluant CH₂Cl₂ Révélation I₂ Rf = 0,27
Spectre R.M.N. 1H : conforme

| Analyse élémentaire : | | | | |
|---|---|---|---|---|
| | % C | % H | % N | % F |
| Théorique | 44,11 | 5,11 | 1,77 | 40,9 |
| Expérimentale | 43,5 | 5,21 | 1,72 | 40,88 |

L'analyse confirme l'obtention de produit attendu.

### Exempel 4 : Synthèse du 6-N-hexadécyloxycarbonylamino-hexanoate de 2-F-octyl éthyle

### Mode opératoire :

Dans un ballon de 250 ml équipé d'un appareil de Dean Stark surmonté d'un réfrigérant sont introduits 14 g (0,03 mole) de 2-F-Octyl Ethanol, 12 g (0,03 mole) de l'acide 6-hexadécyloxycarbonylamino-hexanoïque, ainsi que 90 ml de toluène en présence de 1,2 g d'acide paratoluène sulfonique. Le mélange réactionnel est porté 16 h au reflux du toluène (130°C). Ensuite, 1/3 du toluène est évaporé à l'évaporateur rotatif et 10 g de silice 60H sont ajoutés dans le ballon, l'ensemble est chauffé 5 mn à 80°C puis filtré sur fritté n°4 et rincé avec 30 ml de toluène à 80°C.
Le filtrat cristallise dans la fiole à vide et après retour complet à la température ambiante, le précipité est filtré à son tour sur fritté n°4. Le solide blanc obtenu est séché sous-vide puis recristallisé dans 50 ml d'éther isopropylique.
m = 16,5 g ; rendement = 65 %

### Analyses :

F (Mettler FP85) = 86°C
CCM : Gel de silice 60, éluant : CH₂Cl₂, Révélation I₂ : Rf: 0,42
Spectres R.M.N. 1H et C13 conformes

| Analyse élémentaire : | | | | |
|---|---|---|---|---|
| | % C | % H | % O | % F |
| Théorique | 46,87 | 5,72 | 1,66 | 38,19 |
| Expérimentale | 46,97 | 5,86 | 1,72 | 38,22 |

L'analyse confirme l'obtention du produit attendu.

### Exemple 5 : Synthèse du 11-N-docosyloxycarbonylamino-undecanoate de 2-F-octyl éthyle

### Mode opératoire :

Dans un ballon de 250 ml équipé d'un appareil de Dean Stark surmonté d'un réfrigérant sont introduits 9,28 g (0,02 mole) de 2-F-octyl éthanol, 11,1 g (0,02 mole) de l'acide 11-hexadécyloxycarbonylamino-undecanoïque, ainsi que 60 ml de toluène en présence de 0,57 g d'acide paratoluènesulfonique. Le milieu réactionnel est chauffé pendant 16 heures à 130°C. Ensuite, 6 g de silice 60H sont ajoutés à 80°C, cette température est maintenue pendant 5 mn, puis le mélange est filtré sur fritté n°4 et rincé avec 20 ml de toluène chaud. Le filtrat cristallise dans la fiole à vide, il est filtré sur fritté n°4, séché sous-vide et recristallisé dans 45 ml d'éther isopropylique.
m = 15 g d'une poudre blanche; rendement : 77 %

### Analyses :

F (FP85 METTLER) : 97°C
CCM gel de silice 60, éluant CH₂Cl₂, Révélation I₂, Rf: 0,56
Spectre R.M.N. 13C : conforme

| Analyse élémentaire : | | | | |
|---|---|---|---|---|
| | % C | % H | % N | % F |
| Théorique | 52,85 | 7,06 | 1,4 | 32,3 |
| Expérimentale | 54 | 7,69 | 1,42 | 31,35 |

L'analyse confirme l'obtention du produit attendu.

### Exemple 6 : Synthèse du 8-N-hexadécyloxycarbonylamino-octanoate de 2-F-octyl éthyle

### Mode opératoire :

Dans un ballon de 250 ml équipé d'un appareil de Dean Stark surmonté d'un réfrigérant sont introduits 8,65 g (0,02 mole) de 2-F-octyl éthanol, 9,28 g (0,02 mole) de l'acide 6-hexadécyloxycarbonylamino-octanoïque ainsi que 60 ml de toluène et 0,57 g d'acide paratoluènesulfonique. Le milieu réactionnel est chauffé pendant 16 heures au reflux du toluène (130°C). Ensuite, le toluène est concentré d'un tiers, puis 6 g de silice 60H sont ajoutés dans le ballon.
L'ensemble est chauffé 5 mn à 80°C, puis filtré sur fritté n°4 et rincé avec 20 ml de toluène chaud. Le filtrat cristallise dans la fiole à vide et après retour à température ambiante le précipité est filtré sur fritté n°4. Le solide blanc obtenu (22,6 g) est séché sous-vide puis recristallisé avec 100 ml d'éther isopropylique. Le produit obtenu est une poudre blanche.
m = 10,9 g ; rendement : 63 %

### Analyses :

F (FP85 METTLER) : 92°C
(CCM) gel de silice 60, éluant CH₂Cl₂, Révélation I₂, Rf: 0,47
Spectre R.M.N. 13C : conforme

| Analyse élémentaire : | | | | |
|---|---|---|---|---|
| | % C | % H | % N | % F |
| Théorique | 48,11 | 6 | 1,6 | 36,96 |
| Expérimentale | 47,33 | 5,82 | 1,58 | 37,8 |

### Exemple 7 : Effet épaississant de l'exemple 1 sur différents supports

| Exemple 1 | 1 % | 0,5% | 0,2% |
|---|---|---|---|
| Eutanol G | gel transparent | gel transparent | gel transparent |
| Perhydrosqualène | gel transparent | gel transparent | Pas d'effet |
| Vaseline | gel transparent | gel transparent | Pas d'effet |
| Tournesol | gel transparent | gel transparent | Pas d'effet |
| Parleam | gel transparent | gel transparent | Pas d'effet |
| Huile d'olive | gel transparent | gel transparent | Pas d'effet |

Cet essai montre qu'il est possible selon la nature des milieux huileux à épaissir d'utiliser le 11-N-hexadécyloxycarbonylamino-undécanoate de 2-F-octyl éthyle à des concentrations extrêmement faibles. On a ainsi obtenu des milieux gélifiés. Ces milieux épaissis ont montré une parfaite stabilité sur un recul supérieur à 6 mois.

### Exemple 8 : Rouge à lèvres

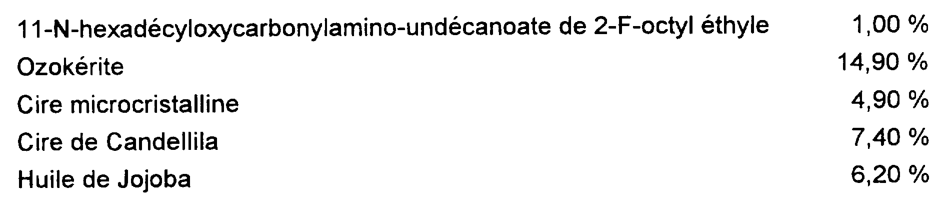

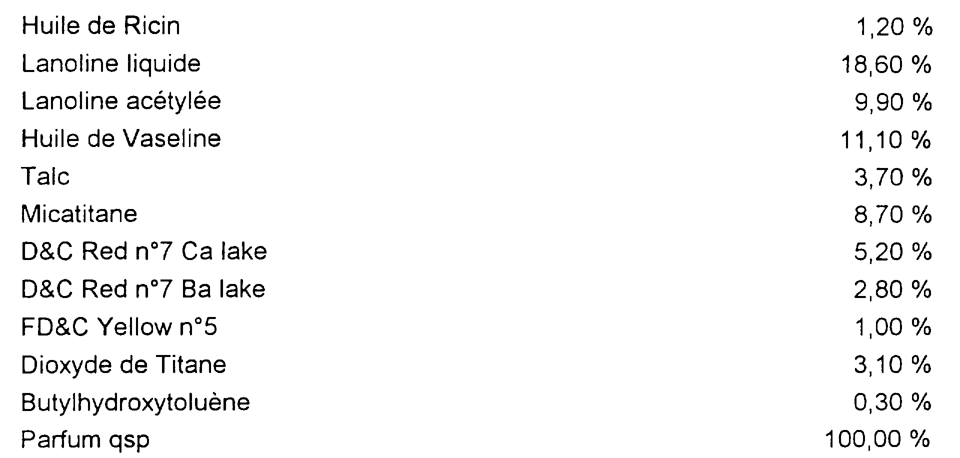

Ce rouge à lèvres se caractérise par de bonnes propriétés d'application et une bonne tenue.

### Exemple 9 : fond de teint

| | |
|---|---|
| 11-N-hexadécyloxycarbonylamino-undécanoate de 2-F-octyl éthyle | 0,50 % |
| Poly diméthylsiloxane | 29,40 % |
| Cetyldiméthicone copolyol ('Abil EM 90' de Goldschmidt) | 3,00 % |
| Eau | 57,00 % |
| Sulfate de magnésium | 0,70 % |
| Glycérol | 5,00 % |
| Oxyde de fer jaune | 0,60 % |
| Oxyde de fer rouge | 0,39 % |
| Oxyde de fer noir | 0,11 % |
| Dioxyde de titane | 2,90 % |
| Conservateurs qsp | 100,00 % |

Ce fond de teint se caractérise aussi par de bonnes propriétés d'application et une bonne tenue cosmétique.

## Revendications

1. Composé fluoré répondant à la formule générale (I) : 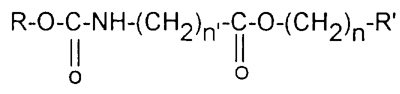 dans laquelle R représente un radical hydrocarboné linéaire ou ramifié, ayant de 8 à 22 atomes de carbone, R' représente un radical perfluorocarboné, linéaire ou ramifié, ayant de 4 à 20 atomes de carbone, n est compris entre 0 et 4, et n' est compris entre 1 et 11.

2. Composé fluoré selon la revendication 1, dans lequel R est un radical hydrocarboné, linéaire ou ramifié, ayant de 8 à 16 atomes de carbone.

3. Composé fluoré selon l'une quelconque des revendications précédentes, dans lequel R' est un radical perfluorocarboné, linéaire ou ramifié, ayant de 4 à 10 atomes de carbone.

4. Composé fluoré selon l'une quelconque des revendications précédentes, choisi dans le groupe constitué par :
le 11-N-hexadécyloxycarbonylamino-undécanoate de 2-F-octyl éthyle ;
le 11-N-hexadécyloxycarbonylamino-undécanoate de 2-F-hexyl éthyle ;
le 2-N-hexadécyloxycarbonylamino-éthanoate de 2-F-octyl éthyle ;
le 2-N-hexadécyloxycarbonylamino-éthanoate de 2-F-hexyl éthyle ;
le 6-N-hexadécyloxycarbonylamino-hexanoate de 2-F-octyl éthyle ;
le 6-N-hexadécyloxycarbonylamino-hexanoate de 2-F-hexyl éthyle ;
le 11-N-docosyloxycarbonylamino-undécanoate de 2-F-octyl éthyle ;
le 11-N-docosyloxycarbonylamino-undécanoate de 2-F-hexyl éthyle ;
le 8-N-hexadécyloxycarbonylamino-octanoate de 2-F-octyl éthyle et
le 8-N-hexadécyloxycarbonylamino-octanoate de 2-F-hexyl éthyle.

5. Procédé de préparation des composés fluorés selon la revendication 1, caractérisé par le fait que l'on fait réagir dans une réaction d'estérification classique, en présence d'un solvant et en milieu acide, un composé de la famille des acides ω-N-alkyloxycarbonylamino-alkylénecarboxyliques, répondant à la formule 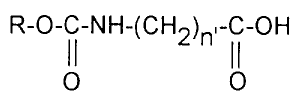 avec un alcool fluoré répondant à la formule :
R'-(CH₂)ₙ-OH
dans lesquelles R représente un radical hydrocarboné linéaire ou ramifié, ayant de 8 à 22 atomes de carbone, R' représente un radical perfluorocarboné, linéaire ou ramifié, ayant de 4 à 20 atomes de carbone, n est compris entre 0 et 4, et n' est compris entre 1 et 11.

6. Procédé de préparation des composés fluorés selon la revendication 1, caractérisé par le fait que l'on fait réagir dans une réaction d'estérification classique, en présence d'un solvant et éventuellement en catalyse basique, un dérivé activé des acides de la famille des ω-N-alkyloxycarbonylamino-alkylénecarboxyliques, répondant à la formule : 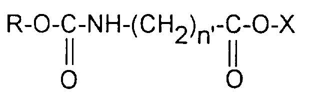 avec un alcool fluoré répondant à la formule :
R'-(CH₂)ₙ-OH
dans lesquelles R représente un radical hydrocarboné linéaire ou ramifié, ayant de 8 à 22 atomes de carbone, R' représente un radical perfluorocarboné, linéaire ou ramifié, ayant de 4 à 20 atomes de carbone, n est compris entre 0 et 4, n' est compris entre 1 et 11, et X représente un groupement activant.

7. Procédé de préparation des composés fluorés selon la revendication 6, dans lequel X est choisi parmi le chlore, le fluor, un groupement azole comme l'imidale ou un groupement OCOOR", dans lequel R" est un radical hydrocarboné ayant de 2 à 4 atomes de carbone.

8. Procédé de préparation des composés fluorés selon l'une quelconque des revendications 6 et 7, dans lequel X est le chlore.

9. Procédé selon l'une quelconque des revendications 5 à 8, dans lequel R est un radical hydrocarboné, linéaire ou ramifié, ayant de 8 à 16 atomes de carbone.

10. Procédé selon l'une quelconque des revendications 5 à 9, dans lequel R' est un radical perfluorocarboné, linéaire ou ramifié, ayant de 4 à 10 atomes de carbone.

11. Utilisation d'au moins un composé fluoré de formule I en tant qu'agent épaississant de milieux non aqueux.

12. Utilisation selon la revendication 11, dans laquelle le composé fluoré est employé à une concentration comprise entre 0,01% et 25% en poids par rapport au poids total du milieu.

13. Utilisation selon l'une quelconque des revendications Il à 12, dans laquelle le composé fluoré est employé à une concentration comprise entre 0,1% et 10% en poids par rapport au poids total du milieu.

14. Utilisation selon l'une quelconque des revendications 11 à 13, dans laquelle le milieu non aqueux est un liquide huileux.

15. Utilisation selon l'une quelconque des revendications 11 à 14, appliquée au domaine de la cosmétique, de la pharmacie, des peintures et vernis, des lubrifiants, des combustibles et de l'industrie alimentaire.

16. Milieu non aqueux comprenant au moins un composé fluoré de formule I.

17. Milieu non aqueux comprenant au moins un composé fluoré de formule I en tant qu'agent épaississant.

18. Milieu non aqueux épaissi selon la revendication 17, comprenant au moins un composé fluoré de formule I à une concentration comprise entre 0,01% et 25% en poids par rapport au poids total du milieu.

19. Milieu non aqueux épaissi selon l'une quelconque des revendications 17 ou 18, comprenant au moins un composé de formule I en une proportion comprise entre 0,1% et 10% en poids par rapport au poids total du milieu.

20. Milieu non aqueux épaissi selon l'une quelconque des revendications 17 à 19, destiné à la cosmétique, à la pharmacie, aux peintures et vernis, aux lubrifiants, aux combustibles et à l'industrie alimentaire.

21. Composition comprenant un milieu non aqueux selon l'une quelconque des revendications 16 à 20.

22. Composition selon la revendication 21, destinée à la cosmétique, à la pharmacie, aux peintures et vernis, aux lubrifiants, aux combustibles et à l'industrie alimentaire.

23. Composition cosmétique ou pharmaceutique selon la revendication 22, se présentant sous la forme d'émulsions, de sticks, de laits ou de vernis.

## Patentansprüche

1. Fluorierte Verbindungen der allgemeinen Formel I 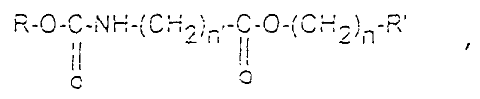 in der bedeuten:
R eine geradkettige oder verzweigte Kohlenwasserstoffgruppe mit 8 bis 22 Kohlenstoffatomen,
R' eine geradkettige oder verzweigte Perfluorkohlenstoffgruppe mit 4 bis 20 Kohlenstoffatomen,
n 0 bis 4
und
n' 1 bis 11.

2. Fluorierte Verbindungen nach Anspruch 1, in deren Formel R eine geradkettige oder verzweigte Kohlenwasserstoffgruppe mit 8 bis 16 Kohlenstoffatomen bedeutet.

3. Fluorierte Verbindungen nach einem der vorhergehenden Ansprüche, in deren Formel R' eine geradkettige oder verzweigte Perfluorkohlenstoffgruppe mit 4 bis 10 Kohlenstoffatomen bedeutet.

4. Fluorierte Verbindungen nach einem der vorhergehenden Ansprüche, die ausgewählt sind unter:
2-Fluoroctylethyl-11-N-hexadecyloxycarbonylamino-undecanoat;
2-Fluorhexylethyl-11-N-hexadecyloxycarbonylamino-undecanoat;
2-Fluoroctylethyl-2-N-hexadecyloxycarbonylamino-ethanoat;
2-Fluorhexylethyl-2-N-hexadecyloxycarbonylamino-ethanoat;
2-Fluoroctylethyl-6-N-hexadecyloxycarbonylamino-hexanoat;
2-Fluorhexylethyl-6-N-hexadecyloxycarbonylamino-hexanoat;
2-Fluoroctylethyl-11-N-docosyloxycarbonylamino-undecanoat;
2-Fluorhexylethyl-11-N-docosyloxycarbonylamino-undecanoat;
2-Fluoroctylethyl-8-N-hexadecyloxycarbonylamino-octanoat und
2-Fluorhexylethyl-8-N-hexadecyloxycarbonylamino-octanoat.

5. Verfahren zur Herstellung der fluorierten Verbindungen nach Anspruch 1, dadurch gekennzeichnet, daß in einer herkömmlichen Veresterungsreaktion in Gegenwart eines Lösungsmittels und in einem sauren Medium eine Verbindung aus der Gruppe der ω-N-Alkyloxycarbonylamino-alkylencarbonsäuren der Formel 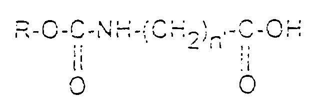 mit einem fluorierten Alkohol der Formel
R'-(CH₂)ₙ-OH,
wobei bedeuten:
R eine geradkettige oder verzweigte Kohlenwasserstoffgruppe mit 8 bis 22 Kohlenstoffatomen,
R' eine geradkettige oder verzweigte Perfluorkohlenstoffgruppe mit 4 bis 20 Kohlenstoffatomen,
n 0 bis 4
und
n' 1 bis 11,
umgesetzt wird.

6. Verfahren zur Herstellung der fluorierten Verbindungen nach Anspruch 1, dadurch gekennzeichnet, daß in einer herkömmlichen Veresterungsreaktion in Gegenwart eines Lösungsmittels und ggfs. unter basischer Katalyse ein aktives Derivat von Säuren aus der Gruppe der ω-N-Alkyloxycarbonylamino-alkylencarbonsäuren der Formel 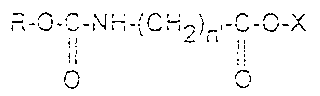 mit einem fluorierten Alkohol der Formel
R'-(CH₂)ₙ-OH
wobei bedeuten:
R eine geradkettige oder verzweigte Kohlenwasserstoffgruppe mit 8 bis 22 Kohlenstoffatomen,
R' eine geradkettige oder verzweigte Perfluorkohlenstoffgruppe mit 4 bis 20 Kohlenstoffatomen,
n 0 bis 4
n' 1 bis 11
und
X eine aktivierende Gruppe,
umgesetzt wird.

7. Verfahren zur Herstellung der fluorierten Verbindungen nach Anspruch 6, wobei X unter Chlor, Fluor, Azolgruppen, wie einer Imidazolgruppe, oder Gruppen OCOOR", wobei R" eine Kohlenwasserstoffgruppe mit 2 bis 4 Kohlenstoffatomen bedeutet, ausgewählt ist.

8. Verfahren zur Herstellung der fluorierten Verbindungen nach Anspruch 6 oder 7, wobei X Chlor bedeutet.

9. Verfahren nach einem der Ansprüche 5 bis 8, wobei R eine geradkettige oder verzweigte Kohlenwasserstoffgruppe mit 8 bis 16 Kohlenstoffatomen bedeutet.

10. Verfahren nach einem der Ansprüche 5 bis 9, wobei R' eine geradkettige oder verzweigte Perfluorkohlenstoffgruppe mit 4 bis 10 Kohlenstoffatomen bedeutet.

11. Verwendung mindestens einer fluorierten Verbindung der Formel I als Verdickungsmittel für nichtwäßrige Medien.

12. Verwendung nach Anspruch 11, wobei die fluorierte Verbindung in einer Konzentration von 0,01 bis 25 Gew.-%, bezogen auf das Gesamtgewicht des Mediums, eingesetzt wird.

13. Verwendung nach Anspruch 11 oder 12, wobei die fluorierte Verbindung in einer Konzentration von 0,1 bis 10 Gew.-%, bezogen auf das Gesamtgewicht des Mediums, eingesetzt wird.

14. Verwendung nach einem der Ansprüche 11 bis 13, wobei das nichtwäßrige Medium eine ölige Flüssigkeit ist.

15. Verwendung nach einem der Ansprüche 11 bis 14 in Anwendung auf das Gebiet der Kosmetik, der Pharmazie, der Farben und Lacke, der Schmiermittel, der Brennstoffe und der Lebensmittelindustrie.

16. Nichtwäßriges Medium, das mindestens eine fluorierte Verbindung der Formel I enthält.

17. Nichtwäßriges Medium, das mindestens eine fluorierte Verbindung der Formel I als Verdickungsmittel enthält.

18. Nichtwäßriges Medium mit Verdickungsmittelgehalt nach Anspruch 17, das mindestens eine fluorierte Verbindung der Formel I in einer Konzentration von 0,01 bis 25 Gew.-%, bezogen auf das Gesamtgewicht des Mediums, enthält.

19. Nichtwäßriges Medium mit Gehalt an Verdickungsmittel nach Anspruch 17 oder 18, das mindestens eine Verbindung der Formel I in einem Mengenanteil von 0,1 bis 10 Gew.-%, bezogen auf das Gesamtgewicht des Mediums, enthält.

20. Nichtwäßriges Medium mit Gehalt an Verdickungsmittel nach einem der Ansprüche 17 bis 19, das zur Anwendung in der Kosmetik, der Pharmazie, für Farben und Lacke, für Schmiermittel, für Brennstoffe sowie in der Lebensmittelindustrie bestimmt ist.

21. Zusammensetzung, die ein nichtwäßriges Medium nach einem der Ansprüche 16 bis 20 enthält.

22. Zusammensetzung nach Anspruch 21, die zur Anwendung in der Kosmetik, der Pharmazie, für Farben und Lacke, für Schmiermittel, für Brennstoffe sowie in der Lebensmittelindustrie bestimmt ist.

23. Kosmetische oder pharmazeutische Zusammensetzungen nach Anspruch 22, die in Form von Emulsionen, Sticks, Milchen oder Lacken vorliegen.

## Claims

1. Fluorinated compound corresponding to the general formula (I): 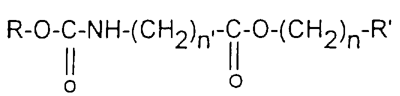 in which R represents a linear or branched hydrocarbon radical having from 8 to 22 carbon atoms, R' represents a linear or branched perfluorocarbon radical having from 4 to 20 carbon atoms, n is from 0 to 4, and n' is from 1 to 11.

2. Fluorinated compound according to Claim 1, in which R is a linear or branched hydrocarbon radical having from 8 to 16 carbon atoms.

3. Fluorinated compound according to either of the preceding claims, in which R' is a linear or branched perfluorocarbon radical having from 4 to 10 carbon atoms.

4. Fluorinated compound according to any one of the preceding claims, chosen from the group consisting of:
2-(F-Octyl)ethyl 11-(N-hexadecyloxycarbonyl)-aminoundecanoate;
2-(F-Hexyl)ethyl 11-(N-hexadecyloxycarbonyl)-aminoundecanoate;
2-(F-Octyl)ethyl 2-(N-hexadecyloxycarbonyl)-aminoethanoate;
2-(F-Hexyl)ethyl 2-(N-hexadecyloxycarbonyl)-aminoethanoate;
2-(F-Octyl)ethyl 6-(N-hexadecyloxycarbonyl)-aminohexanoate;
2-(F-Hexyl)ethyl 6-(N-hexadecyloxycarbonyl)-aminohexanoate;
2-(F-Octyl)ethyl 11-(N-docosyloxycarbonyl)-aminoundecanoate;
2-(F-Hexyl)ethyl 11-(N-docosyloxycarbonyl)-aminoundecanoate;
2-(F-Octyl)ethyl 8-(N-hexadecyloxycarbonyl)-aminooctanoate and
2-(F-Hexyl)ethyl 8-(N-hexadecyloxycarbonyl)-aminooctanoate.

5. Process for the preparation of the fluorinated compounds according to Claim 1, characterized in that a compound from the family of ω-(N-alkyloxycarbonyl)aminoalkylenecarboxylic acids, corresponding to the formula: 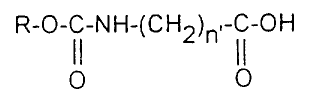 is reacted, in a conventional esterification reaction, in the presence of a solvent and in acid medium, with a fluorinated alcohol corresponding to the formula:
R'-(CH₂)ₙ-OH
in which R represents a linear or branched hydrocarbon radical having from 8 to 22 carbon atoms, R' represents a linear or branched perfluorocarbon radical having from 4 to 20 carbon atoms, n is from 0 to 4, and n' is from 1 to 11.

6. Process for the preparation of the fluorinated compounds according to Claim 1, characterized in that an activated derivative from the family of ω-(N-alkyloxycarbonyl)aminoalkylenecarboxylic acids, corresponding to the formula: 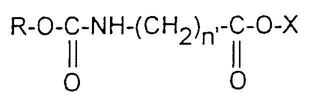 is reacted, in a conventional esterification reaction, in the presence of a solvent and optionally in basic catalysis, with a fluorinated alcohol corresponding to the formula:
R'-(CH₂)ₙ-OH
in which R represents a linear or branched hydrocarbon radical having from 8 to 22 carbon atoms, R' represents a linear or branched perfluorocarbon radical having from 4 to 20 carbon atoms, n is from 0 to 4, n' is from 1 to 11, and X represents an activating group.

7. Process for the preparation of the fluorinated compounds according to Claim 6, in which X is chosen from chlorine, fluorine, an azole group, such as imidazole, or a group OCOOR", in which R" is a hydrocarbon radical having from 2 to 4 carbon atoms.

8. Process for the preparation of the fluorinated compounds according to either of Claims 6 and 7, in which X is chlorine.

9. Process according to any one of Claims 5 to 8, in which R is a linear or branched hydrocarbon radical having from 8 to 16 carbon atoms.

10. Process according to any one of Claims 5 to 9, in which R' is a linear or branched perfluorocarbon radical having from 4 to 10 carbon atoms.

11. Use of at least one fluorinated compound of formula (I) as defined in any one of claims 1 to 4 as thickening agent for non-aqueous media.

12. Use according to Claim 11, in which the fluorinated compound is employed at a concentration of from 0.01 % to 25 % by weight with respect to the total weight of the medium.

13. Use according to either of Claims 11 and 12, in which the fluorinated compound is employed at a concentration of from 0.1 % to 10 % by weight with respect to the total weight of the medium.

14. Use according to any one of Claims 11 to 13, in which the non-aqueous medium is an oily liquid.

15. Use according to any one of Claims 11 to 14, applied to the field of cosmetics, pharmaceuticals, paints and varnishes, lubricants, fuels and the food industry.

16. Non-aqueous medium comprising at least one fluorinated compound of formula I as defined in any one of claims 1 to 4.

17. Non-aqueous medium comprising at least one fluorinated compound of formula I as defined in any one of claims 1 to 4 as thickening agent.

18. Thickened non-aqueous medium according to Claim 17, comprising at least one fluorinated compound of formula I at a concentration of from 0.01 % to 25 % by weight with respect to the total weight of the medium.

19. Thickened non-aqueous medium according to either of Claims 17 and 18, comprising at least one compound of formula I in a proportion of from 0.01 % to 10 % by weight with respect to the total weight of the medium.

20. Thickened non-aqueous medium according to any one of Claims 17 to 19, intended for cosmetics, pharmaceuticals, paints and varnishes, lubricants, fuels and the food industry.

21. Composition comprising a non-aqueous medium according to any one of Claims 16 to 20.

22. Composition according to Claim 21, intended for cosmetics, pharmaceuticals, paints and varnishes, lubricants, fuels and the food industry.

23. Cosmetic or pharmaceutical composition according to Claim 22 which is provided in the form of emulsions, sticks, milks or varnishes.
